# EUROPEAN PATENT SPECIFICATION

(11) **EP 2 709 570 B1**
(45) Date of publication and mention of the grant of the patent: **13.04.2016**
(21) Application number: 12720179.6
(22) Date of filing: 10.05.2012
(51) Int. Cl.: A61F 5/14, A43B 7/22, A43B 7/14

(54) **AN ORTHOTIC INSOLE**
ORTHOPÄDISCHE EINLAGE
SEMELLE ORTHÉTIQUE

(30) Priority: 17.05.2011 GB 201108203
(43) Date of publication of application: 26.03.2014
(73) Proprietor: Supple, Emma Lucy, Enfield, Middlesex EN2 6HD (GB)
(72) Inventor: Supple, Emma Lucy, Enfield, Middlesex EN2 6HD (GB)
(74) Representative: Dolleymores
(86) International application number: PCT/EP2012/058620
(87) International publication number: WO 2012/156267

(56) References cited:
- DE-C- 895 045
- US-A- 2 821 032
- US-A- 3 828 792
- US-A- 5 069 212
- US-A1- 2008 217 816

## Description

The present invention relates to an orthotic insole and in particular to an orthotic insole that can be inserted into an existing shoe. However, an orthotic insole in accordance with the present invention may also be used as an integral part of the construction of a shoe.

Available designs of insoles, orthotics and arch supports work on the premise that the instep, medical longitudinal arch, or natural arch of the foot requires support and this is achieved by physically filling the natural arch of the foot. The instep is therefore fully supported by one or more of a variety of materials, which control and support the foot function by infill of the arches. These devices are tolerated, but may take time to adjust to the foot. Also due to the necessity of width and length they are sometimes difficult to accommodate into footwear.

Known orthotic insoles are disclosed, for example, in U2821032, US2008/0217816 A1, and DE895045. In particular, an orthotic insole according to the preamble of appended claim 1 is disclosed in US3828792.

It is an object of the present invention to provide an alternative form of orthotic insole.

It is a further object of the invention to provide an insole which facilitates and improves effective foot motion

It is a further object of the invention to provide an insole which facilitates improved weight bearing of the foot instep.

It is a further object of the invention to provide an insole which facilitates optimal (and normal) functioning of the plantar fascia (plantar aponeurosis) tendon including the "windlass mechanism", a podiatric term to denote the "winding up" of the deep flexor tendon as the first metatarso phalangeal joint - big toe - bends into a 90° position for propulsion and "toe off" stage in gait. The invention seeks to aid this propulsive movement by enabling and not obstructing the medial longitudinal arch component.

Still another object of the invention is to provide an insole which can more easily fit into footwear and improve shoe wearer comfort.

According to the present invention there is provided an orthotic insole as disclosed in claim 1, namely comprising a cupped heel plate and an extension portion extending forward of the heel plate and terminating in the region of the talonavicular junction on the plantar aspect of the foot, the extension portion being cut away in both a lateral portion and a medial portion, so that the extension portion forms a central spur.

The term "cut away" is used to describe the shape of the insole and does not relate to the method of forming the "cut away" portions.

The insole will preferably be designed for either the right or left foot, wherein the medial portion is more extensively cut away than the lateral portion.

The present invention provides an insole which may be inserted into a shoe or which alternatively may be integral with the structure of the shoe. Where the insole is to be inserted in a shoe the heel plate is generally configured in the shape of a cupped heel base to the calcaneum area, so that the centrally placed extension of the insole extends to the soft tissue junction of the talar navicular joint and is retained in place below the talar navicular joint by the shape of the heel portion being retained in place by the shoe and the wearer heel. An insole in accordance with the present invention supports and facilitates motion and alignment of the arches of the foot by the application of a dorsiflexory force, a force applied in an upward direction, to a defined central area whilst facilitating medial and lateral movement of the foot by only primarily supporting the central portion of the calcaneum (heel bone) and plantar aponeurosis (plantar fascia) from its origin at the calcaneum tuberosity.

The plantar fascia tendon has three distinct segments; medial, central and lateral. For normal gait patterns the requirement is for the foot to pass through the fulcrum of force and momentum in a centrally, symmetrically manner for the most appropriate mechanics, because normal foot motion requires a balance between pronation and supination throughout the gait or walking cycle, where any deviance to the lateral is referred to as supination and any deviance to the medial is referred to as pronation.

In anatomical skeletal terms the central ligament of the plantar fascia has its origin from the calcaneus (heel bone) and the three segments to the plantar fascia run under the transverse arch consisting of cuboid, navicular and cuneiforms. The plantar fascia then inserts into the distal phalanges of the toes as tendon slips.

Other muscles involved in the functioning of the foot are Quadratus Plantae, flexor hallucis longus, the peroneal tendons and abducto hallucis longus and tibialis posterior.

When a human biped is walking or running the foot arches supported by the tendon and ligament complex normally facilitate the central support and fulcrum for forward propulsion. Along with ankle movement (plantarflexion and dorsiflexion) sub talar joint motion (tri-planar and then onto mid-tarsal joint movement culminating in the propulsive toe-off phase). A complex tri-planar movement.

By wearing shoes with varying heel heights the normal foot function can be impaired. The lesser metatarsal arch has to load for longer time sections as the shoe front loads the foot mechanics. By taking into account the requirement for dorsiflexory force in this defined area, the plantar fascia and central soft tissue anatomical positions can be enhanced.

Impaired biomechanical foot function, not related to pathological excess pronation (hyperpronation) or supination leads to excess forces loading prematurely on to the lesser metatarsals heads and increases tendencies for the formation of callosities on the plantar aspect of the ball of the foot, buckling or hammering of lesser toes as the plantar fascia insertions are involved prematurely and excessively in the gait cycle.

By employing the present invention, the central portion of soft tissues and skeletal structures is supported facilitating improved effective foot motion and improved weight bearing of the foot instep whilst permitting normal functioning of the plantar fascia tendon including the "windlass mechanism". Also by placing a supportive uplift to the central portion of the soft tissue, the skeletal structures terminating at the talonavicular joint, an insole in accordance with the present invention results in the ease of pressure on the distal aspects of the weight bearing aspects of the metatarsals.

The cut away portions of the insole, preferably with more insole material on the medial side being absent than on the lateral side, facilitates the normal motion of the central ligaments and the transverse keystone arch of the foot.

The insole may be formed from a polypropylene material and advantageously comprises a single homogenous structure. The composition of the insole may comprise copper, known to have healing properties. The material of the insole is preferably a semi-rigid material.

It is advantageous if the distal end of the extension portion of the insole curves downwards, such that the extension portion forms an arch with a void below the arch, thereby permitting the extension portion to flex when inserted into a shoe and weight is applied to the upper surface of the insole, permitting a more constant force to be applied by the extension portion to the foot than would be the case if the there was not a void below the extension portion.

Preferably, the insole is dimensioned such that 80% of the area of the insole has a thickness of between 3 and 5 mm, enabling it to be accommodated in a shoe and to provide the required rigidity. Advantageously, a central portion in the length direction of the extension portion, is between 40% and 60% of the maximum width of the insole. Also it is preferable that the medial portion is cut away by more than 25%, but less than 40% of the maximum width of the insole and/or that the lateral portion is cut away by more 15% but less than 30% of the maximum width of the insole.

The insole preferably has a total length of between 100 and 150 mm, such that it terminates in the region of the talonavicular junction of the foot of a wearer.

The present invention will now be described, by way of example only, with reference to the accompanying drawings, of which:
Figure 1A is a plan view of an insole in accordance with the present invention;
Figure 1B is a side elevation of the insole of Figure 1a;
Figure 2 illustrates the positioning of the insole relative to the plantar surface of a right foot;
Figure 3 shows the positioning of the insert relative to the plantar ligaments of a right foot; and
Figure 4 illustrates the positioning of the insole relative to the plantar fascia of a left foot.

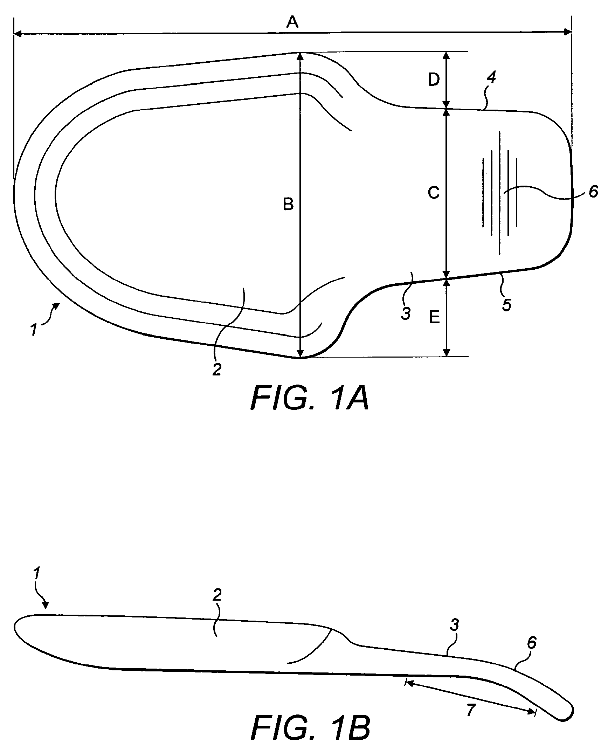

Referring to Figure 1A, an insole, indicated generally as 1, in accordance with the present invention is preferably constructed as a homogenous semi-flexible to rigid plastic polymer, for example polypropylene, to which copper may be added for its known medical properties. The insole comprises a heel plate 2, preferably in the shape of a heel cup base to the calcaneum area, with a central extension portion extending forward of the heel plate 2, the central portion 3, being cut away at a lateral portion 4 and medial portion 5 so that the extension portion 3 is in the form of a central band.

The distal end 6 of the extension portion curves downwards in the region 6 so that its lower surface forms an arch in the region 7 which arch can be compressed when weight is applied to the upper surface of the insole 1.

The insole 1 has a length A preferably between 100 and 150 mm. The insole 1 has a maximum width B in the heel plate region of between 50 to 70 mm, with the insole having a thickness of preferably between 3 and 5 mm over at least 80% of its area.

The extension portion 3 at a point midway between the widest point of the insole and the distal end of the extension portion has a width C of between 40% and 60% of a maximum width B of the insole. At this point, the medial portion is preferably cut away by an amount D greater than 25%, but less than 40% of the maximum width B of the insole. The lateral portion is preferably cut away at this point by a width E of more than 15%, but less than 30% of the maximum width B of the insole.

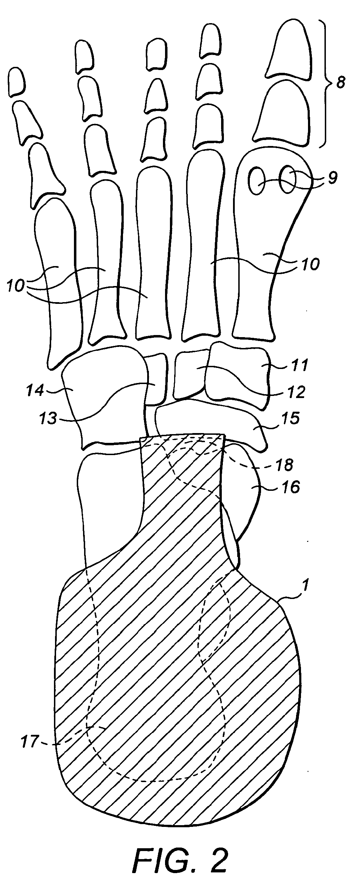

Referring to Figure 2, there is illustrated a plantar view of the bones of the foot comprising the phalanges 8, sesamoids 9, metatarsals 10, the medial cuneiform 11, the intermediate cuneiform 12, the lateral cuneiform 13, cuboid 14, navicular 15, talus 16 and calcaneum 17. The insole 1 is shown superimposed on the plantar supporting the calcaneum 17 and extending to the region of the talus navicular joint 18.

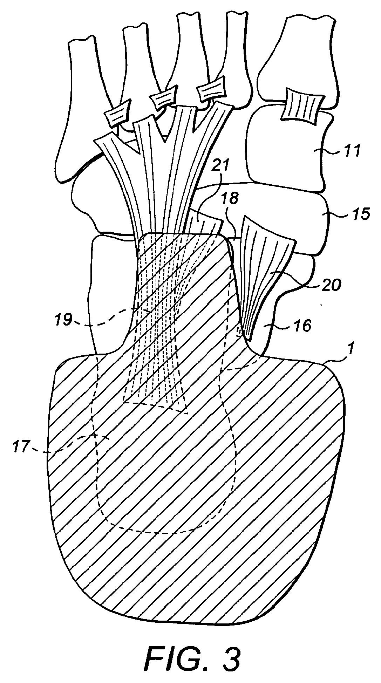

Figure 3 illustrates the plantar ligaments of the foot comprising the long plantar ligament 19, the plantar calcaneonavicular ligament (the spring ligament) 20 and the short plantar ligament 21. The insole 1 is shown superimposed over the plantar ligaments and is seen to extend to the region of the talar navicular junction 18 in the region below the long plantar and short plantar ligaments.

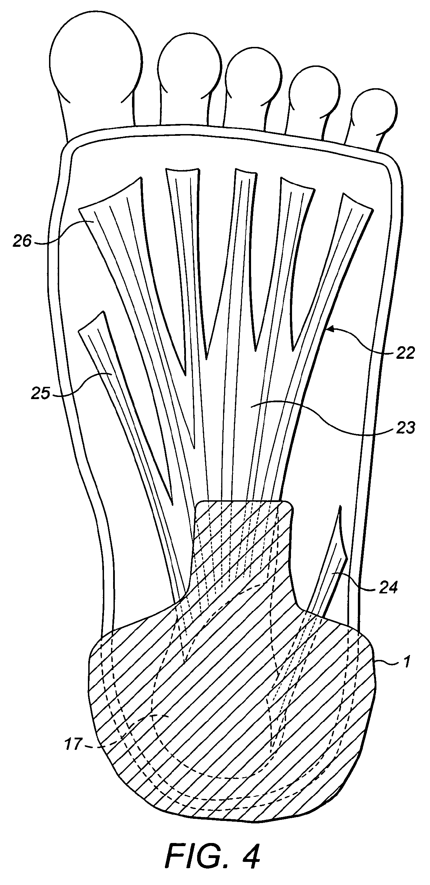

Referring to Figure 4, there is illustrated the position of the insole 1 relative to the plantar fascia, indicated generally as 22 and comprising a central portion 23, a lateral portion 24 and a medial portion 25. The lateral portion 23 being inserted into the digital slips in the region 26.

From the figures the insole can be seen to support the central portion of the plantar fascia distal to its insertion to the calcaneum, terminating at the talar navicular joint. Just proximal to the ascending transverse arch.

Support the central portion of the plantar aspect of the foot distal to the calcaneum as the arch commences, causes the underlying bone structure to be supported at the moment it is in a plantarflexory motion hence enabling optimal foot function.

Supporting the ligaments as illustrated causes the ligaments to be supported at the biomechanical moment of transfer of weight from heel strike to midfoot loading.

Supporting the plantar fascia in the region of the central band of the plantar fascia cause the medial and lateral bands of the plantar fascia to work without impingement from orthotic material and facilitate first and fifth ray functionality.

The absence of the extension portion 3 in the lateral and medial portions permits the first ray (first metatarsal and cuneiform section) and the lateral ray (5^{th} and 4^{th} metatarsal complexes) to move without impingement from orthotic material.

The absence of the medial and lateral portions of the insole permits the deep ligaments to the first ray (first metatarsal and cuneiform section) and the lateral ray (5^{th} and 4^{th} metatarsal complexes) to move without impingement from orthotic material.

This arrangement is advantageous because it permits normal plantarflexion and dorsiflexion movement of the first ray mechanics (first metatarsal and cuneiform section) and the lateral ray (5^{th} and 4^{th} metatarsal complexes) without impingement from orthotic material (in individual optimal mode). It also permits active engagement of the lesser metatarsals and the first ray on the pivot point to be centrally supported by the insole.

The insole has been illustrated in the previous drawings by way of example as a separate insole to be inserted into a shoe. However, a shoe may comprise such an insert within the construction of the shoe.

## Claims

1. An orthotic insole comprising a cupped heel plate with an extension portion extending forward of the heel plate, the extension portion being cut away in both a lateral portion and a medial portion so that the extension portion forms a central spur,
**characterised in that** the extension portion is configured to terminate in the region of the talonavicular junction on the plantar aspect of the foot.

2. An insole as claimed in Claim 1, wherein the medial portion is more extensively cut away than the lateral portion.

3. An insole as claimed in Claim 1 or Claim 2 formed from polypropylene.

4. An insole as claimed in any preceding claim, comprising a single homogenous structure.

5. An insole as claimed in any preceding claim, comprising copper in the composition of the insole.

6. An insole as claimed in any preceding claim, comprising a semi-rigid material.

7. An insole as claimed in any preceding claim, wherein the distal end of the extension portion curves downwards such that the extension portion forms an arch with a void below the arch, to permit the extension portion to flex when inserted in a shoe and weight is applied to its upper surface.

8. An insole as claimed in any preceding claim, wherein 80% of the area of the insole has a thickness of between 3mm and 5mm.

9. An insole as claimed in any preceding claim, wherein a central portion in the length direction of the extension portion is between 40% and 60% of the maximum width of the insole.

10. An insole as claimed in any preceding claim, wherein the medial portion is cut away by more than 25% but less than 40% of the maximum width of the insole.

11. An insole as claimed in any preceding claim, wherein the lateral portion is cut away by more than 15% but less than 30% of the maximum width of the insole.

12. An insole as claimed in any preceding claim, having a length of between 100mm and 150mm.

13. An insole as claimed in any preceding claim, arranged to be inserted into a shoe and retained in place by the external shape of the heel portion of the insole engaging with the sides of the shoe in the region of the heel of the shoe.

14. A shoe with an insole as claimed in any one of claims 1 to 12 fabricated as integral part of the construction of the shoe.

## Patentansprüche

1. Eine orthopädische Einlage bestehend aus einer schalenförmigen Fersenplatte mit einer von der Fersenschale nach vorne verlaufenden Verlängerung,
wobei die Verlängerung sowohl in einem lateralen Bereich als auch einem medialen Bereich zurückgeschnitten ist, so dass die Verlängerung eine zentrale Leiste bildet,
**dadurch gekennzeichnet, dass** die Verlängerung so konfiguriert ist, dass sie im Bereich des Talonavikular-Gelenks auf der Sohlenfläche des Fußes endet.

2. Eine Einlage entsprechend Anspruch 1, wobei der mediale Bereich mehr als der laterale Bereich zurückgeschnitten ist.

3. Eine Einlage entsprechend Anspruch 1 oder Anspruch 2, die aus Polypropylen geformt ist.

4. Eine Einlage entsprechend einem der vorhergehenden Ansprüche, die aus einer einzigen homogenen Struktur besteht.

5. Eine Einlage entsprechend einem der vorhergehenden Ansprüche, die in der Zusammensetzung der Einlage Kupfer enthält.

6. Eine Einlage entsprechend einem der vorhergehenden Ansprüche, die aus einem halbflexiblen Material besteht.

7. Eine Einlage entsprechend einem der vorhergehenden Ansprüche, wobei sich das distale Ende der Verlängerung nach unten biegt, so dass die Verlängerung einen Bogen mit einem Hohlraum unter dem Bogen bildet, damit sich die Verlängerung beim Einlegen in einen Schuh und Belasten der Oberfläche biegen kann.

8. Eine Einlage entsprechend einem der vorhergehenden Ansprüche, wobei 80% der Einlagenfläche eine Dicke von zwischen 3 mm und 5 mm aufweist.

9. Eine Einlage entsprechend einem der vorhergehenden Ansprüche, wobei ein mittlerer Teil in Längsrichtung der Verlängerung zwischen 40% bis 60% der Maximalbreite der Einlage beträgt.

10. Eine Einlage entsprechend einem der vorhergehenden Ansprüche, wobei der mediale Bereich mehr als 25%, jedoch weniger als 40% der maximalen Breite der Einlage zurückgeschnitten ist.

11. Eine Einlage entsprechend einem der vorhergehenden Ansprüche, wobei der laterale Bereich mehr als 15%, jedoch weniger als 30% der maximalen Breite der Einlage zurückgeschnitten ist.

12. Eine Einlage entsprechend einem der vorhergehenden Ansprüche, die eine Länge von zwischen 100 mm und 150 mm aufweist.

13. Eine Einlage entsprechend einem der vorhergehenden Ansprüche, die so angelegt ist, dass sie in einen Schuh eingelegt und in Position gehalten wird, indem die externe Form des Fersenteils sich mit den Seiten des Schuhs in der Ferse des Schuhs verbindet.

14. Ein Schuh mit einer Einlage entsprechend einem der Ansprüche 1 bis 12, die als integraler Bestandteil der Schuhkonstruktion erstellt wurde.

## Revendications

1. Semelle orthopédique comprenant une plaque de talon en forme de cupule dotée d'une partie d'extension qui s'étend en avant de la plaque de talon, la partie d'extension présentant une découpe à la fois dans une portion latérale et une partie médiane de sorte que la partie d'extension forme un éperon central,
**caractérisée en ce que** la partie d'extension est configurée pour se terminer dans la région de l'articulation astragalo-scaphoïdienne sur la face plantaire du pied.

2. Semelle orthopédique selon la revendication 1, dans laquelle la découpe est plus importante dans la partie médiane que dans la partie latérale.

3. Semelle orthopédique selon la revendication 1 ou la revendication 2 formée à partir de polypropylène.

4. Semelle orthopédique selon l'une quelconque des revendications précédentes, qui comprend une structure homogène unique.

5. Semelle orthopédique selon l'une quelconque des revendications précédentes, qui comprend du cuivre dans la composition de la semelle.

6. Semelle orthopédique selon l'une quelconque des revendications précédentes, qui comprend un matériau semi-rigide.

7. Semelle orthopédique selon l'une quelconque des revendications précédentes, dans laquelle l'extrémité distale de la partie d'extension est courbée vers le bas de sorte que la partie d'extension forme une voûte sous laquelle est ménagé un vide permettant le fléchissement de la partie d'extension lors de son insertion dans une chaussure et à l'application d'un poids sur sa surface supérieure.

8. Semelle orthopédique selon l'une quelconque des revendications précédentes, dans laquelle 80 % de la semelle a une épaisseur qui est entre 3 mm et 5 mm.

9. Semelle orthopédique selon l'une quelconque des revendications précédentes, dans laquelle la largeur d'une portion centrale, dans la direction de longueur, de la partie d'extension est entre 40 % et 60 % de la largeur maximale de la semelle.

10. Semelle orthopédique selon l'une quelconque des revendications précédentes, dans laquelle la partie médiane est découpée sur plus de 25 % mais moins de 40 % de la largeur maximale de la semelle.

11. Semelle orthopédique selon l'une quelconque des revendications précédentes, dans laquelle la partie latérale est découpée sur plus de 15 % mais moins de 30 % de la largeur maximale de la semelle.

12. Semelle orthopédique selon l'une quelconque des revendications précédentes, dont la longueur est entre 100 mm et 150 mm.

13. Semelle orthopédique selon l'une quelconque des revendications précédentes, qui est configurée pour être insérée dans une chaussure et maintenue en place par la forme externe de la partie de talon de la semelle accouplée aux côtés de la chaussure dans la région du talon de la chaussure.

14. Chaussure dotée d'une semelle orthopédique selon l'une quelconque des revendications 1 à 12 qui est fabriquée en tant que partie intégrale de la construction de la chaussure.
